# EUROPEAN PATENT APPLICATION

(11) **EP 3 534 153 A1**
(43) Date of publication of application: **04.09.2019**
(21) Application number: 17865465.3
(22) Date of filing: 30.10.2017
(51) Int. Cl.: G01N 27/00, C12Q 1/68, G01N 33/543

(54) **MEASURING METHOD AND MEASURING SYSTEM**

(30) Priority: 31.10.2016 JP 2016212608
(71) Applicant: Miraca Research Institute G.K., Tokyo 192-0031 (JP)
(72) Inventor: MATSUNO, Tatsuki, Tokyo 163-0410 (JP); HIKI, Shinichiro, Tokyo 163-0410 (JP); OKA, Akihiro, Tokyo 163-0410 (JP)
(74) Representative: Pfenning, Meinig & Partner mbB
(86) International application number: PCT/JP2017/039057
(87) International publication number: WO 2018/079761

(57) **Abstract**

[Problem] To provide a measuring method and a measuring system capable of improving accuracy of measurement.

[Solution] The measuring method is a measuring method of measuring a measurement target contained in a sample, including a pass-through step of passing a complex 78, formed by allowing the measurement target to react with a predetermined carrier, through a pore 41 provided in a substrate 40 by electrophoresis, etc., a measuring step of measuring a size of the complex 78 based on change in electrical characteristics occurring when the complex 78 is passed through the pore 41 in the pass-through step, and an determining step of determining the number of measurement targets based on the size of the complex 78 measured in the measuring step.

## Description

### Technical Field

The present invention relates to a measuring method and a measuring system.

### Background Art

Conventionally, a method has been proposed as a technology for measuring the number of biomolecules contained in a sample. In the method, a biomolecule binds to a magnetic particle in a specific reaction, the binding biomolecule binds to a fluorescently labeled probe molecule in a specific reaction, a plurality of such complex is immobilized on a supporting substrate, the number of fluorescent molecules generated from the immobilized ones (specifically, probe molecules) is detected, and the detected number is measured as the number of biomolecules (for example, see Patent Document 1).

### Citation List

### Patent Document

Patent Document 1: International Publication No. 2013/051651

### Summary of the Invention

### Technical Problem

However, in the conventional method, in the case of detecting the fluorescence, for example, in a case in which a probe molecule bound to a magnetic particle by a nonspecific reaction is present on a supporting substrate, this probe molecule is detected as noise signal. Therefore, it is difficult to accurately measure the number of biomolecules bound to the magnetic particle and the probe molecule by a specific reaction, and thus there is room for improvement from the viewpoint of improving the accuracy of measurement.

The invention has been made to solve the above-mentioned problems in the conventional technology, and an object of the invention is to provide a measuring method and a measuring system capable of improving the accuracy of measurement.

### Solution to Problem

In order to solve the above mentioned problem and achieve the purpose, a measuring method of claim 1 is a measuring method of measuring a measurement target contained in a sample, the method comprising: a pass-through step: passing a complex formed by allowing the measurement target to react with a predetermined carrier through a pore provided in a substrate; a measuring step: measuring a size of the complex based on change in electrical characteristics occurring when the complex is passed through the pore in the pass-through step; and a determining step: determining the number of measurement targets based on the size of the complex measured in the measuring step.

The measuring method of claim 2 according to claim 1, wherein the complex comprises the measurement target, a first carrier capable of binding to the measurement target, and a second carrier capable of binding to the measurement target, and in the determining step, the number of measurement targets for each complex passing through the pore in the pass-through step is determined based on the size of the complex measured in the measuring step, and the integrated value of each determined number is determined as the total number of measurement targets.

The measuring method of claim 3 according to claim 1, wherein the complex comprises the measurement target, a first carrier capable of bind to the measurement target, and a second carrier capable of bind to the measurement target, the measuring method further comprises a dissociating step of forming a dissociated complex by dissociating the first carrier from the complex before the pass-through step, the dissociated complex formed in the dissociating step is passed through the pore in the pass-through step, a size of each dissociated complex is measured in the measuring step based on change in electrical characteristics occurring when the dissociated complex is passed through the pore in the pass-through step, and the number of measurement targets is determined in the determining step based on the size of each dissociated complex measured in the measuring step.

A measuring system of claim 4 is a measuring system for measuring a measurement target contained in a sample, the measuring system comprising: a substrate in which a pore is formed; pass-through means for passing through to the pore a complex formed by allowing the measurement target to react with a predetermined carrier; measuring means for measuring a size of the complex based on change in electrical characteristics occurring when the complex is passed through the pore by the pass-through means; and determining means for determining the number of measurement targets based on the size of each complex measured by the measuring means.

The measuring system of claim 5 according to claim 4, wherein the complex comprises the measurement target, a first carrier capable of binding to the measurement target, and a second carrier capable of binding to the measurement target, and the measuring system further comprises complex information storage means for storing complex information configured by mutually associating an information indicating the size of the complex and number-of-measurement targets information indicating the number of measurement targets with each other, and the determining means determines the number of measurement targets of each complex passed through the pore by the pass-through means based on the complex information stored in the complex information storage means and the size of the complex measured by the measuring means, and determines an integrated number of each determined number as the number of measurement targets.

The measuring system of claim 6 according to claim 4, wherein the complex comprises the measurement target, a first carrier capable of binding to the measurement target, and a second carrier capable of binding to the measurement target, the measuring system further comprises dissociating means for forming a dissociated complex by dissociating the first carrier from the complex, the pass-through means passes the dissociated complex formed by the dissociating means through the pore, the measuring means measures a size of the dissociated complex based on change in electrical characteristics occurring when the dissociated complex is passed through the pore by the pass-through means, and the determining means determines the number of measurement targets based on the size of the dissociated complex measured by the measuring means.

According to the measuring method of claim 1 and the measuring system of claim 4, the pass-through step of passing the complex through the pore by electrophoresis, and the like, the measuring step of the size of the complex based on the change in electrical characteristics occurring when the complex is passed through the pore in the pass-through step, and the determining step of the number of measurement targets based on the size of the complex measured in the measuring step are included. Thus, when compared to the conventional technology (the technology for measuring the number of measurement targets by detecting fluorescence of the fluorescently labeled probe molecules), the number of complexes is determined based on the size information of the complex rather than the number information of fluorescent substances. Thus, for example, even in a case in which a plurality of complexes (that is, the measurement target bound to the carrier in the specific reaction) and a plurality of carriers nonspecifically bound only to the probe molecules are passed through the pore, it is possible to accurately measure the number of complexes. Therefore, it is possible to improve the accuracy of measurement.

According to the measuring method of claim 2 and the measuring system of claim 5, the complex includes the measurement target, the first carrier, and the second carrier. In the determining step, the number of measurement targets for each complex passing through the pore in the pass-through step is determined based on the size of the complex measured in the measuring step. The integrated value of the determined numbers is determined as the number of measurement targets. Thus, it is possible to accurately measure the number of measurement targets bound to the first carrier and the second carrier in the specific reaction, and to further improve accuracy of measurement. In addition, no particular and complicated procedure is required in each step, and thus it is possible to speed up and simplify measurement procedure.

According to the measuring method of claim 3 and the measuring system of claim 6, the complex includes the measurement target, the first carrier, and the second carrier, and the dissociating step of forming the dissociated complex by dissociating the first carrier from the complex is included before the pass-through step. Further, the dissociated complex formed in the dissociating step is passed through the pore by electrophoresis, etc. in the pass-through step, the size of the dissociated complex is measured in the measuring step based on the change in electrical characteristics occurring when the dissociated complex is passed through the pore in the pass-through step, and the number of measurement targets is determined in the determining step based on the size of the dissociated complex measured in the measuring step. Therefore, only the number of the measurement target bound to the second carrier in the specific reaction is measured and therefore it is possible to further improve accuracy of measurement. In addition, since the size of the dissociated complex is less variable than the size of the complex, it is easier to determine the number of measurement targets in the determining step, so that it is possible to further improve accuracy of measurement.

### Brief Description of the Drawings

Fig. 1 is a schematic diagram illustrating a measuring system according to a first embodiment of the invention.
Fig. 2 is a diagram illustrating a configuration example of a complex table.
Fig. 3 is a schematic diagram illustrating an overview of a first reaction step.
Fig. 4 is a schematic diagram illustrating an overview of a second reaction step, Fig. 4(a) is a diagram illustrating a state before adding a second carrier bound to a second carrier-side antibody, and Fig. 4(b) is a diagram illustrating a state after adding the second carrier bound to the second carrier-side antibody.
Fig. 5 is a schematic diagram illustrating an overview of a pass-through step, Fig. 5(a) is a diagram illustrating a state before a complex is passed through a pore, and Fig. 5(b) is a diagram illustrating a state after the complex is passed through the pore.
Fig. 6 is a schematic diagram illustrating an overview of a dissociating step according to a second embodiment.
Fig. 7 is an enlarged view of an area A of Fig. 6, Fig. 7(a) is a diagram illustrating a state in which a first carrier is dissociated from a complex, and Fig. 7(b) is a diagram illustrating a state in which a dissociated complex has been formed.
Fig. 8 is a schematic diagram illustrating an overview of a pass-through step, Fig. 8(a) is a diagram illustrating a state before the dissociated complex is passed through the pore, and Fig. 8(b) is a diagram illustrating a state after the dissociated complex has been passed through the pore.
Fig. 9 is a graph showing an experimental result of Example 1 (measuring method according to the first embodiment) and is a graph showing an experimental result for each specimen size related to a first specimen and an experimental result for each specimen size related to a second specimen.
Fig. 10 is a table showing an experimental result of Example 2 (measuring method according to the second embodiment) and is a table showing experimental results for a first specimen to a fifth specimen.

### Mode for Carrying Out the Invention

Hereinafter, a description is given of embodiments of a measuring method and a measuring system according to this invention with reference to accompanying drawings. First, [I] a basic concept of the embodiments is described, and next [II] specific contents of the embodiments are described, and finally [III] modifications to the embodiments are described. However, the invention is not limited by the embodiments.

### [1] Basic concept of embodiments

First, the basic concept of the embodiments is described. The embodiments generally relate to the measuring method and the measuring system for measuring a measurement target contained in a sample.

Here, the "sample" means a material to be examined or analyzed, and contains the measurement target and a solvent in the embodiments. In addition, the "measurement target" means a substance corresponding to a target of measurement among substances contained in the sample, and includes for example, an antigen, a biomolecule, protein, nucleic acid, a low molecular weight, etc. In addition, the "solvent" means a material having a largest amount (number of molecules) among materials contained in the sample, and includes, for example, a reaction fluid capable of causing the measurement target to react with a predetermined carrier and capable of passing a current therethrough (for example, an antigen-antibody reaction solution, etc.). Here, the "predetermined carrier" means a particle as a base to which the measurement target is immobilized and includes a first carrier and a second carrier in the embodiments. Among these carriers, the "first carrier" means a particle that can bind to the measurement target, and includes, for example, a magnetic particle, a resin, a carbon material, an inorganic substance, an organic substance, an alloy, etc. In addition, the "second carrier" means a particle which can bind to the measurement target and is preferably smaller than the first carrier, and includes, for example, a gold nanoparticle, a fluorescent dye, a magnetic particle, a resin, a carbon material, an inorganic substance, an organic substance, an alloy, etc. A bond between the measurement target and the first carrier (or the second carrier) may be a direct bond between the measurement target and a particle, but preferably be an indirect bond in which a probe molecule, an antibody, binding protein, or the like is immobilized on the first carrier (or the second carrier) in advance and the bond to the measurement target is formed via the probe molecule, the antibody, or the like. In addition, this measuring method may be of any type, and may be chosen from for example, a one-step competitive method, a one-step sandwich method, a delay one-step method, a two-step sandwich method, a diluted two-step method, and the like. Hereinafter, in the embodiments, as an example, a description is given of the case of measuring the measurement target using the two-step sandwich method by setting the measurement target to an antigen, setting the solvent to an antigen-antibody reaction solution, setting the first carrier to a magnetic particle bound to antibody (hereinafter referred to as a "first carrier-side antibody") in advance, setting the second carrier to a nanoparticle bound to antibody (hereinafter referred to as a "second carrier-side antibody") in advance, and setting a complex to a product formed by the first carrier, the first carrier-side antibody, the measurement target, the second carrier-side antibody, and the second carrier.

In addition, with regard to a measuring method according to a first embodiment, schematically, 1) a "first reaction step" of adding a first carrier immobilized with a first carrier-side antibody to a sample so that a measurement target contained in the sample reacts with the first carrier-side antibody (antigen-antibody reaction), thereby binding the measurement target to the first carrier, 2) a "second reaction step" of adding a second carrier immobilized with a second carrier-side antibody to the sample so that the measurement target bound to the first carrier-side antibody in the first reaction step reacts with the second carrier-side antibody (antigen-antibody reaction), thereby binding the measurement target to the second carrier, 3) a "pass-through step" of causing a complex formed in the second reaction step (that is, the first carrier, the first carrier-side antibody, the measurement target, the second carrier-side antibody, and the second carrier) to pass through a pore described below, 4) a "measuring step" of a size of the complex passing through the pore described below in the pass-through step, and 5) an "determining step" of the number of molecules of measurement targets based on the size of the complex measured in the measuring step are successively performed.

In addition, with regard to a measuring method according to a second embodiment, schematically, after 1) a "first reaction step" and 2) a "second reaction step" are successively performed similarly to the measuring method according to the first embodiment, 3) a "dissociating step" of forming a dissociated complex by dissociating a first carrier from a complex formed in the second reaction step, 4) a "pass-through step" of causing the dissociated complex formed in the dissociating step to pass through a pore described below, 5) a "measuring step" of measuring a size of the dissociated complex passing through the pore described below in the pass-through step, and 6) an "determining step" of determining the number of molecules of measurement targets based on the size of the dissociated complex measured in the measuring step are successively performed. Here, the "dissociated complex" includes, for example, a complex including a first carrier-side antibody, a measurement target, a second carrier-side antibody, and a second carrier (hereinafter, referred to as a "first dissociated complex"), a complex only including a first carrier-side antibody (hereinafter, referred to as a "second dissociated complex"), and the like.

### [II] Specific contents of embodiments

Next, specific contents of the embodiments are described.

### [First embodiment]

First, the first embodiment is described. The first embodiment corresponds to an aspect in which the number of molecules of measurement targets is determined based on a size of a complex.

### (Configuration)

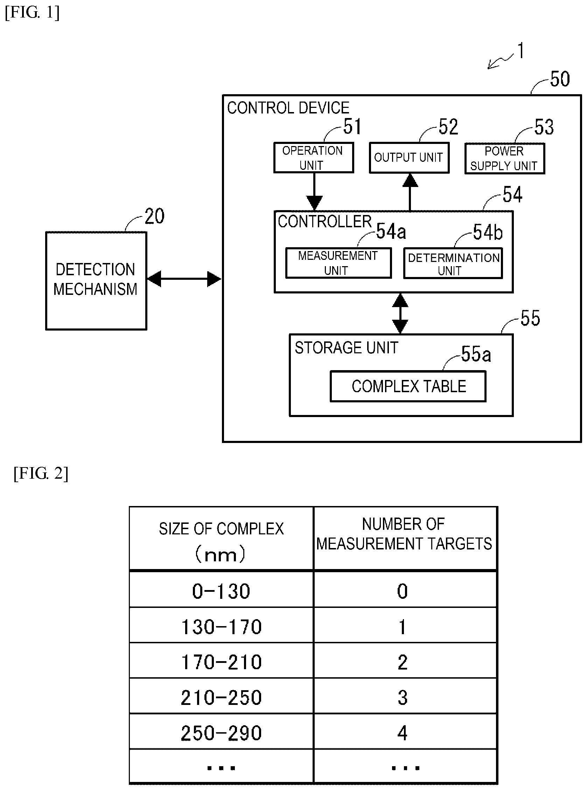

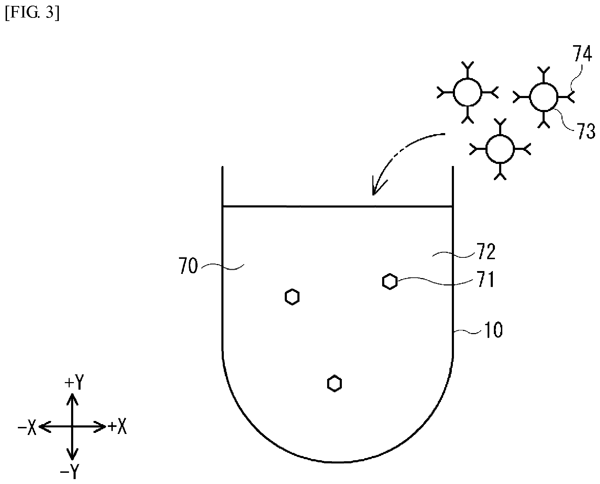

First, a description is given of a configuration of a measuring system according to the first embodiment. Fig. 1 shows a schematic diagram illustrating the measuring system according to the first embodiment of this invention. As illustrated in Fig. 1, a measuring system 1 is roughly composed of a reaction chamber 10 of Fig. 3 described below, a detection mechanism 20, and a control device 50. In addition, among these components, each of a first electrode portion described below, a second electrode portion described below, and a detector described below of the detection mechanism 20 is electrically connected to the control device 50 via wiring (not illustrated).

### (Configuration - reaction chamber)

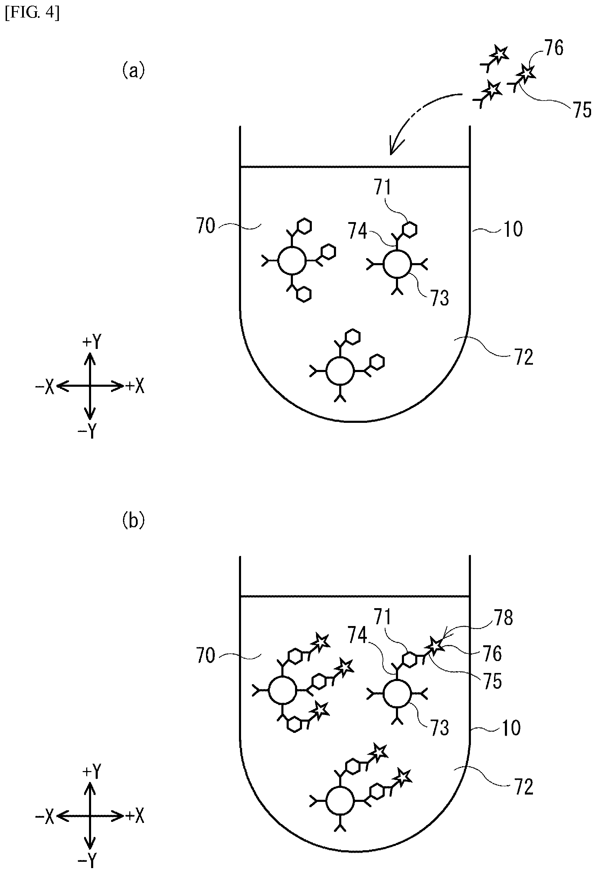

The reaction chamber 10 of Fig. 3 described below is a container for producing a complex 78 of Fig. 4(b) described below, is configured with, for example, a known reaction chamber, etc. formed of a glass material, a resin material, ceramics (silicon nitride, silicon chloride, etc.), a metal material, an inorganic material, an organic material etc., and is provided in the vicinity of the detection mechanism 20.

### (Configuration - detection mechanism)

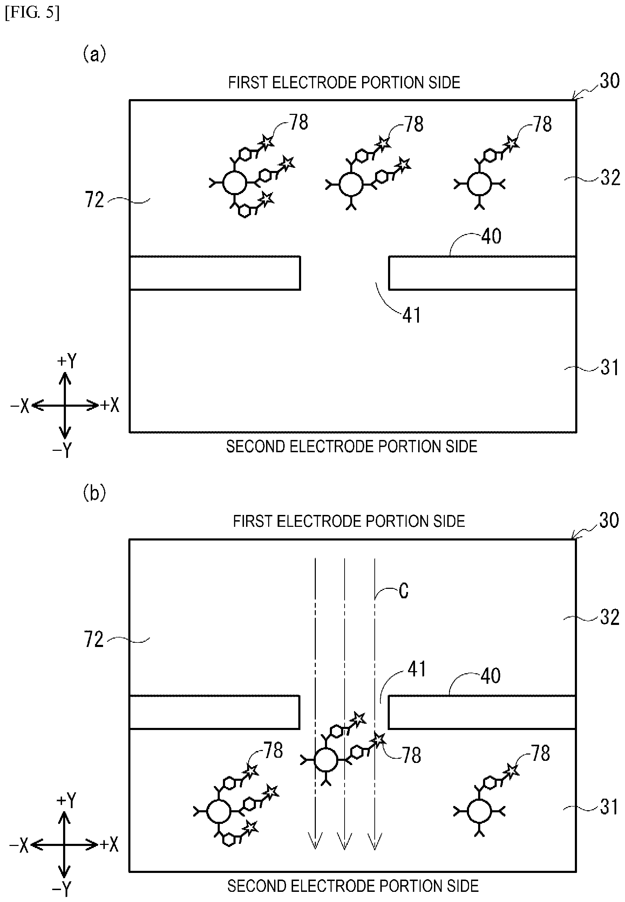

Returning to Fig. 1, the detection mechanism 20 is a mechanism for detecting change in electrical characteristics (for example, a change in current, etc.) in an accommodating portion 30 of Fig. 5 described below, and includes a placing table (not illustrated), the accommodating portion 30, a substrate 40 of Fig. 5 described below, a pass-through portion (not illustrated), and a detector (not illustrated).

### (Configuration - detection mechanism - placing table)

The placing table is a table on which the accommodating portion 30 is placed, is configured with, for example, a known placing table, etc., and is placed on an installation surface (not illustrated).

### (Configuration - detection mechanism - accommodating portion)

The accommodating portion 30 of Fig. 5 is an accommodating means for the substrate 40 and includes a lower accommodating portion 31 and an upper accommodating portion 32. The lower accommodating portion 31 is formed in a hollow shape having an open upper surface and is placed on an upper surface of the placing table. The upper accommodating portion 32 is formed in a hollow shape having an open lower surface and is provided such that an open portion of the upper accommodating portion 32 (a lower end portion of the upper accommodating portion 32) is covered by the lower accommodating portion 31.

### (Configuration - detection mechanism - substrate)

The substrate 40 is a plate for partitioning the lower accommodating portion 31 and the upper accommodating portion 32 of the accommodating portion 30. For example, the substrate 40 is formed of a plate-like body having any planar shape (for example, a cross shape, a rectangular shape, a circular shape, etc.), and is provided substantially horizontally such that at least a part of the substrate 40 is accommodated in the accommodating portion 30. A thickness of the substrate 40 is preferably equal to or less than a diameter of a pore 41 described below. For example, a substrate whose thickness is thinner than a diameter of a pore such as a low aspect ratio pore described in Tsutsui et al., ACS NANO Vol. 6, No. 4, pp. 3499-3505 (2012) may be used. By reducing the thickness of the substrate 40, it is possible to more accurately analyze a state (size, shape, etc.) of a particle passing through, and to measure the number of molecules of measurement targets present on the particle with higher accuracy.

In addition, as illustrated in Fig. 5 described below, the pore 41 is formed in the substrate 40. The pore 41 is a through-hole which the complex 78 passes through. This pore 41 is formed, for example, in a substantially circular shape, and is disposed, for example, in a central portion of the substrate 40. In addition, a specific shape and size of this pore 41 may be any shape and any size, however, in the first embodiment, the pore 41 is set to a shape which is substantially the same as or slightly larger than a maximum diameter of the complex 78 such that the complex 78 can be reliably passed therethrough and detection sensitivity of the detector can be maintained at a predetermined value or more.

In addition, a material of the substrate 40 can be arbitrarily chosen as long as the material is excellent in chemical resistance and does not pass electricity, and can be formed of, for example, a glass material, a resin material, ceramics (silicon nitride, silicon chloride, etc.), a metal material, an inorganic material, an organic material, or the like.

### (Configuration - detection mechanism - pass-through portion)

The pass-through portion is a passing-through means for passing the complex 78 through the pore 41. The passage portion preferably has a configuration of passing the complex 78 through the pore 41 by electrophoresis and includes the first electrode portion and the second electrode portion (neither of which is illustrated).

The first electrode portion is configured with, for example, a known electrode member, etc., provided above the substrate 40, and disposed to be in contact with a solution in the upper accommodating portion 32 of the accommodating portion 30.

The second electrode portion is an electrode portion corresponding to a minus pole or a ground pole when the first electrode portion corresponds to a positive pole, corresponding to a positive pole or a ground pole when the first electrode portion corresponds to a minus pole, and corresponding to a positive pole or a minus pole when the first electrode portion corresponds to a ground pole. The second electrode portion is configured with, for example, a known electrode member, etc., provided below the substrate 40, and disposed to be in contact with a solution in the lower accommodating portion 31.

### (Configuration - detection mechanism - detector)

The detector is a detecting means for change in electrical characteristics (a change in current, etc. in the embodiment) in the accommodating portion 30. The detector is configured with, for example, a known current measurement sensor, etc., provided inside the accommodating portion 30 or outside the accommodating portion 30 and at a position around the accommodating portion 30, and fixed to the accommodating portion 30 (or a supporting member (not illustrated)) by a fixing tool, etc.

### (Configuration - control device)

Returning to Fig. 1, the control device 50 is a device for controlling the measuring system 1, and includes an operation unit 51, an output unit 52, a power supply unit 53, a controller 54, and a storage unit 55 as illustrated in Fig. 1. Incidentally, the control device 50 can be configured with, for example, a known personal computer, etc., and thus a detailed description thereof is omitted.

### (Configuration - control device - operation unit)

The operation unit 51 is an operating means for receiving an operation input related to various types of information. For example, the operation unit 51 is configured with remote operating means such as a touch pad and a remote controller, or a known operating means such as a hard switch.

### (Configuration - control device - output unit)

The output unit 52 is an output means for outputting various types of information based on control of the controller 54, and is configured with, for example, a known display means such as a flat panel display such as a liquid crystal display or an organic EL display, and a known audio output means such as a speaker, etc.

### (Configuration - control device - power supply unit)

The power supply unit 53 is a power supply means for supplying power supplied from a commercial power supply (not illustrated) or power stored in the power supply unit 53 to the pass-through portion and the detector of the detection mechanism 20 and each unit of the control device 50.

### (Configuration - control device - controller)

The controller 54 is a control means for controlling each unit of the control device 50. Specifically, the controller 54 is a computer including a CPU and an internal memory such as a RAM for storing various programs interpreted and executed on the CPU (including a basic control program such as an OS and an application program activated on the OS to realize a specific function), a program, and various types of data.

In addition, as illustrated in Fig. 1, the controller 54 includes a measurement unit 54a and a determination unit 54b with respect to its functional concept. The measurement unit 54a is a measuring means for measuring a size of the complex 78 (specifically, volume of the complex 78) based on change in electrical characteristics occurring when the complex 78 is passed through the pore 41 in a system of Fig. 5. The determination unit 54b is a determining means for determining the number of measurement targets 71 of Fig. 3 described below based on the size of the complex 78 measured by the measurement unit 54a. Incidentally, details of a process executed by the controller 54 are described below. In addition, the "measurement unit 54a" and the "detector" of the detection mechanism 20 correspond to "measuring means" in claims.

### (Configuration - control device - storage unit)

The storage unit 55 is a recording means for recording a program and various types of data necessary for an operation of the control device 50, and is configured with, for example, a hard disk (not illustrated) as an external recording device. However, in place of the hard disk or together with the hard disk, it is possible to use any another recording medium including a magnetic recording medium such as a magnetic disk, an optical recording medium such as a DVD or a Blu-ray disc, or an electrical recording medium such as a flash ROM, a USB memory, or an SD card.

In addition, as illustrated in Fig. 1, the storage unit 55 includes a complex table 55a. The complex table 55a is a complex information storage means for storing information for specifying the complex 78 (hereinafter referred to as "complex information").

Fig. 2 shows a diagram illustrating a configuration example of the complex table 55a. As illustrated in Fig. 2, the complex table 55a is configured by mutually correlating an item "the size of the complex" and an item "the number of measurement targets" with information corresponding to each item. Here, the information corresponding to the item "the size of the complex" is complex size information indicating the size of the complex 78, and corresponds to, for example, "0-130" (which indicates "0 nm or more and less than 130 nm"), etc. which is a size of the complex 78 illustrated in Fig. 2. The information corresponding to the item "the number of measurement targets" is number-of-measurement targets information indicating the number of measurement targets 71, and corresponds to, for example, "0", "1", "2", etc., each of which is the number of measurement targets 71 illustrated in Fig. 2. In the number-of-measurement targets information, the number of measurement targets 71 corresponding to the size of the complex 78 is set to allow determination of the number of measurement targets 71 bound to a first carrier 73 depending on the size of the complex 78 since, for example, the size of the complex 78 in which one measurement target 71 binds to one first carrier 73 is different from the size of the complex 78 in which two (or three) measurement targets 71 bind to one first carrier 73 as illustrated in Fig. 4(a) described below.

### (Measuring method)

Next, a description is given of a measuring method performed using the measuring system 1 configured as described above. Fig. 3 shows a schematic diagram illustrating an overview of the first reaction step. Fig. 4 shows a schematic diagram illustrating an overview of the second reaction step. Fig. 4(a) shows a diagram illustrating a state before adding a second carrier 76 bound to a second carrier-side antibody 75, and Fig. 4(b) shows a diagram illustrating a state after adding the second carrier 76 bound to the second carrier-side antibody 75. Fig. 5 shows a schematic diagram illustrating an overview of the pass-through step. Fig. 5(a) shows a diagram illustrating a state before the complex 78 is passed through the pore 41, and Fig. 5(b) is a diagram illustrating a state after the complex 78 is passed through the pore 41. In the description below, an X direction of Fig. 3 is referred to as a right-left direction of the measuring system 1 (a -X direction is referred to as a leftward direction of the measuring system, and a +X direction is referred to as a rightward direction of the measuring system 1), a Y direction of Fig. 3 is referred to as a vertical direction of the measuring system 1 (a +Y direction is referred to as an upward direction of the measuring system 1, and a -Y direction is referred to as a downward direction of the measuring system 1), and a direction orthogonal to the X direction and the Y direction is referred to as a front-rear direction (a direction leading to a near side of a page of Fig. 3 is referred to as a frontward direction of the measuring system 1, and a direction leading to a far side of the page of Fig. 3 is referred to as a rearward direction of the measuring system 1). As described above, the measuring method according to the first embodiment includes the first reaction step, the second reaction step, the pass-through step, the measuring step, and the determining step. Hereinafter, detailed content of each step is described.

### (Measuring method - first reaction step)

First, the first reaction step is described. The first reaction step is performed in a procedure below to make the measurement target 71 contained in a sample 70 bind to the first carrier 73. In more detail, first, as illustrated in Fig. 3, after the first carrier 73 bound to a first carrier-side antibody 74 is added to the reaction chamber 10 that accommodates the sample 70 including the measurement target 71 and a solvent 72, the sample 70 to which the first carrier 73 is added is agitated. Here, the added first carriers 73 may be of any quantity. For example, the quantity is set so that all the measurement targets 71 contained in the sample 70 can react with the first carrier-side antibody 74 (antigen-antibody reaction). As an example, to improve efficiency of the above reaction, the quantity of the first carriers 73 is set to an excessive quantity with respect to a quantity of the measurement targets 71 (for example, 100 times the quantity of the measurement targets 71, etc.) (incidentally, the same is applied to a quantity of added second carriers 76 in the second reaction step described below). Subsequently, until a predetermined time elapses after the first carrier 73 is added, the agitated sample 70 is left at a predetermined temperature (for example, 37°C). Specifically, the predetermined time is set to a time longer than or equal to a generally assumed time required to cause the measurement target 71 to react with the first carrier-side antibody 74. Subsequently, after the predetermined time elapses, washing is carried out to remove a substance not reacting with the first carrier 73 in the sample 70. A washing method may be any method. For example, in the case of using a magnetic particle as the first carrier 73, in a state in which the first carrier 73 reacting with the measurement target 71 and the first carrier 73 not reacting with the measurement target 71 are magnetically collected in a part of the reaction chamber 10 by a magnet (not illustrated) installed in the part of the reaction chamber 10 (as an example, a side surface portion of the reaction chamber 10, etc.), the substance not reacting with the first carrier 73 in the sample 70 is washed out with the solvent 72, etc. (incidentally, the same is applied to a washing method in the second reaction step described below).

### (Measuring method - second reaction step)

Next, the second reaction step is described. In the second reaction step, a procedure below is performed to make the measurement target 71 reacting in the first reaction step bind to the second carrier 76. In more detail, first, as illustrated in Fig. 4(a), after the second carrier 76 bound to the second carrier-side antibody 75 is added to the reaction chamber 10, the sample 70 to which the second carrier 76 is added is agitated. Subsequently, until a predetermined time elapses after the second carrier 76 is added, the agitated sample 70 is left at a predetermined temperature (for example, 37°C). Specifically, the predetermined time is set to a time longer than or equal to a generally assumed time required to cause the measurement target 71 to react with the second carrier-side antibody 75. Subsequently, after the predetermined time elapses, washing is carried out to remove the second carrier 76 not reacting with the measurement target 71.

### (Measuring method - pass-through step)

Next, the pass-through step is described. The pass-through step is performed in a procedure below to pass the complex 78 illustrated in Fig. 4(b) formed in the second reaction step (the first carrier 73, the first carrier-side antibody 74, the measurement target 71, the second carrier-side antibody 75, and the second carrier 76) through the pore 41 of the substrate 40. In more detail, first, a predetermined amount of liquid comprising the complex 78 and the solvent 72 are removed from the reaction chamber 10 by a liquid feeding pipe (for example, a pipette, etc.) (not illustrated), and the complex 78 and solvent 72 in the liquid are injected into the accommodating portion 30 of the detection mechanism 20 (specifically, into the upper accommodating portion 32 illustrated in Fig. 5(a)). In this case, for example, it is desirable that a solvent 72 having substantially the same component as that of the solvent 72 of the sample 70 is put in a lower storage portion 31 of the accommodating portion 30 in advance. Subsequently, as illustrated in Fig. 5(b), a current C is caused to flow for a predetermined time through the accommodating portion 30 via the first electrode portion and the second electrode portion by the measurement unit 54a of the control device 50 to move the injected complex 78 downward (that is, electrophoresis is used), thereby passing the complex 78 through the pore 41. Specifically, it is preferable that the predetermined time is set to a time longer than or equal to a generally assumed time required for the predetermined number of injected complexes 78 to pass through the pore 41. In this case, the above-described operation is repeatedly performed until the predetermined number of complexes 78 formed in the second reaction step is passed through the pore 41. Alternatively, it is possible to adopt a scheme in which a time is set in advance (for example, 10 minutes) regardless of the number of complexes 78 passing through, and the complexes 78 passing through the pore 41 within the set time are analyzed.

### (Measuring method - measuring step)

Next, the measuring step is described. The measuring step is performed in a procedure below to measure the size of the complex 78. In more detail, first, by the measurement unit 54a of the control device 50, using a known electrical detection method (for example, an electrical nano-pulse method, etc.), the current C flowing through the accommodating portion 30 is detected by the detector at a timing at which the current C starts to flow in the pass-through step, and detection of the current C is continued until the predetermined time during which the current C flows elapses (incidentally, this operation is repeatedly performed until the pass-through step is finished). Here, the "electrical nano-pulse method" refers to a method of obtaining, as a pulse signal, a change in electric resistance occurring when a nanoparticle passes through the pore 41 in an electrified liquid. In addition, the "pulse signal" is a signal indicating a size of the nanoparticle (specifically, the volume of the nanoparticle). For example, a higher pulse signal indicates that a particle is larger. Subsequently, the measurement unit 54a of the control device 50 determines the size of each complex 78 passing through the pore 41 in the pass-through step based on the detected change in the current C (a time history change of the current C) with reference to relationship data which is stored in the storage unit 55 in advance and indicates a relationship between the size of the complex 78 and a magnitude of the current C (for example, data indicating a proportional relationship between the size of the complex 78 and the magnitude of the current C, etc.).

### (Measuring method - determining step)

Next, the determining step is described. The determining step is performed in a procedure below to determine the number of measurement targets 71 contained in the sample 70. In more detail, first, the determination unit 54b of the control device 50 determines the number of measurement targets 71 for each complex 78 passing through the pore 41 in the pass-through step based on complex information stored in the complex table 55a and the size of the complex 78 measured in the measuring step. A method of determining the number of measurement targets 71 may be any method, however, in the first embodiment, number-of-measurement targets information corresponding to the size of the complex 78 measured in the measuring step is extracted from the number-of-measurement targets information of the complex information, and a value of the extracted number-of-measurement targets information is determined as the number of measurement targets 71 of the complex 78. For example, in a case in which the size of the complex 78 measured in the measuring step = 160 nm, when the number-of-measurement targets information = 1 illustrated in Fig. 2 is extracted, the number of measurement targets 71 of the complex 78 = 1 is determined. Subsequently, the determination unit 54b of the control device 50 determines a integrated value of the determined numbers of every complex 78 as the number of measurement targets 71 contained in the sample 70. Subsequently, the controller 54 of the control device 50 causes the output unit 52 to output the determined number of measurement targets 71 contained in the sample 70. A method of outputting the number of measurement targets 71 may be any method, however, for example, the number of measurement targets 71 is displayed on a screen of the output unit 52 (display means). Alternatively, audio data indicating the number of measurement targets 71 (as an example, audio data including a routine message "the number of measurement targets contained in the sample 70 was XXX", etc.) may be audio-output through the output unit 52 (audio output means).

According to the measuring method described above, when compared to a conventional technology (a technology for measuring the number of measurement targets 71 by detecting fluorescence of fluorescently labeled probe molecules), the number of complexes 78 is measured based on the size of the complex 78 rather than measuring the number of fluorescent substances. Thus, for example, even in a case in which a plurality of complexes 78 or a plurality of carriers (as an example, the second carrier 76 bound to the first carrier 73, etc.) is passed through the pore 41, it is possible to accurately measure only the number of complexes 78 (that is, measurement targets 71 bound to the first carrier 73 and the second carrier 76 by a specific reaction). Therefore, it is possible to improve accuracy of measurement. In addition, no particular and complicated work is required in each step, and thus it is possible to speed up and simplify measurement work.

According to the first embodiment, the pass-through step of passing the complex 78 through the pore 41, the measuring step of the size of the complex 78 based on the change in electrical characteristics occurring when the complex 78 is passed through the pore 41 in the pass-through step, and the determining step of the number of measurement targets 71 based on the size of the complex 78 measured in the measuring step are included. Thus, when compared to the conventional technology (the technology for measuring the number of measurement targets by detecting fluorescence of the fluorescently labeled probe molecules), the number of complexes 78 is measured based on the size of the complex 78 rather than measuring the number of fluorescent substances. Thus, for example, even in a case in which a plurality of complexes 78 (that is, the measurement target 71 bound to the carrier by the specific reaction) and a plurality of carriers nonspecifically bound only to the probe molecules is passed through the pore 41, it is possible to accurately measure only the number of complexes 78. Therefore, it is possible to improve accuracy of measurement.

In addition, the complex 78 includes the measurement target 71, the first carrier 73, and the second carrier 76. In the determining step, the number of measurement targets 71 for each complex 78 passing through the pore 41 in the pass-through step is determined based on the size of the complex 78 measured in the measuring step. The integrated value of the determined numbers is determined as the number of measurement targets 71. Thus, it is possible to accurately measure the number of measurement targets 71 bound to the first carrier 73 and the second carrier 76 by the specific reaction, and to further improve accuracy of measurement. In addition, no particular and complicated work is required in each step, and thus it is possible to speed up and simplify measurement work.

### [Second embodiment]

Next, the second embodiment is described. The second embodiment corresponds to a mode in which the number of measurement targets is determined based on a size of a dissociated complex. In the second embodiment, preferably, the first carrier 73 is a particle on which a predetermined linker is immobilized and which can bind to a measurement target through the linker (further through a probe molecule, an antibody, etc. as necessary). Remaining configurations are substantially the same as the configurations of the first embodiment unless specifically noted. Further, the same reference symbols and/or names as those used in the first embodiment is assigned to configurations substantially the same as those of the first embodiment as necessary, and description thereof is omitted.

### (Configuration)

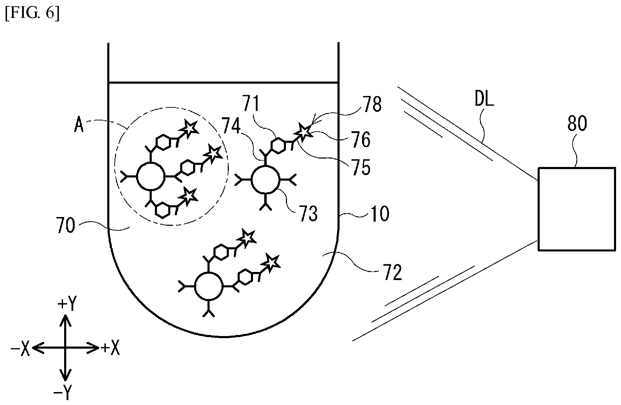

First, a description is given of a configuration of a measuring system according to the second embodiment. The measuring system according to the second embodiment roughly includes a reaction chamber 10 of Fig. 6 described below, a detection mechanism (not illustrated), a dissociation portion 80 of Fig. 6 described below, and a control device (not illustrated) (however, a complex table of the control device is omitted). In addition, among these components, each of a first electrode portion, an electrode portion, and a detector of the detection mechanism, and the dissociation portion 80 is electrically connected to the control device via wiring.

### (Configuration - dissociation portion)

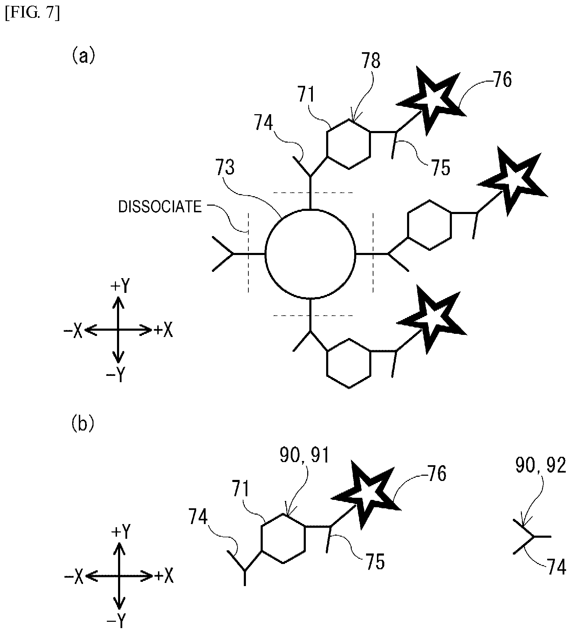

The dissociation portion 80 is a dissociating means for forming a dissociated complex 90 of Fig. 7(b) described below by dissociating the first carrier 73 from the complex 78. The dissociation portion 80 is configured with a light radiation device that radiates light DL (hereinafter referred to as "dissociation light DL") of Fig. 6 described below capable of dissociating the first carrier 73 from the complex 78 by cutting the linker in a case in which the predetermined linker immobilized on the first carrier 73 is a photo-cleavable linker and is provided near the reaction chamber 10. Here, the "dissociation light DL" includes, for example, light having a wavelength at which a photo-cleavage reaction can be caused between the first carrier 73 and the second carrier-side antibody 75 (as an example, extreme ultraviolet rays, vacuum ultraviolet rays, near ultraviolet rays, visible rays, near infrared rays, mid infrared rays, far infrared rays, etc.), etc. As the photo-cleavable linker, for example, it is possible to use any of known photo-cleavable linkers such as 2-nitrobenzyl group, p-hydroxyphenacyl group, (2-nitrophenyl) ethyl group, (coumarin-4-yl) methyl group, etc.

### (Measuring method)

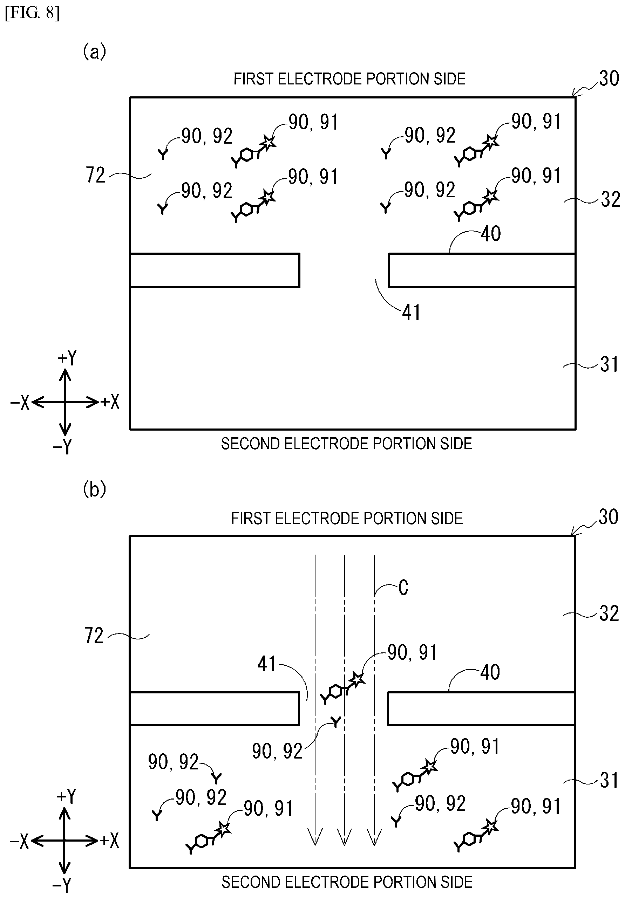

Next, a description is given of a measuring method performed using the measuring system 1 configured as described above. Fig. 6 shows a schematic diagram illustrating an overview of a dissociating step. Fig. 7 shows an enlarged view of an area A of Fig. 6. Fig. 7(a) shows a diagram illustrating a state in which the first carrier 73 is dissociated from the complex 78, and Fig. 7(b) shows a diagram illustrating a state in which the dissociated complex 90 has been formed. Fig. 8 shows a schematic diagram illustrating an overview of a pass-through step. Fig. 8(a) shows a diagram illustrating a state before the dissociated complex 90 is passed through the pore 41, and Fig. 8(b) shows a diagram illustrating a state after the dissociated complex 90 has been passed through the pore 41. As described above, a measuring method according to the second embodiment includes the first reaction step, the second reaction step, the dissociating step, the pass-through step, the measuring step, and the determining step. Incidentally, in this measuring method, the first reaction step and the second reaction step are the same as those of the measuring method according to the first embodiment, and thus a description thereof is omitted.

### (Measuring method - dissociating step)

First, the dissociating step is described. The dissociating step is performed in a procedure below to form the dissociated complex 90 before the pass-through step. In more detail, first, a controller of the control device radiates the dissociation light DL from the dissociation portion 80 as illustrated in Fig. 6 to the complex 78 formed in the second reaction step to dissociate the first carrier 73 from the complex 78 as illustrated in Fig. 7(a), thereby forming the dissociated complex 90 (specifically, a first dissociated complex 91 and a second dissociated complex 92 illustrated in Fig. 7(b)). A method of radiating the dissociation light DL may be any method. However, since it is desirable to perform radiation so that first carriers 73 can be dissociated from all complexes 78 formed in the dissociating step, for example, as illustrated in Fig. 6, the dissociation light DL is radiated to the entire content (that is, the dissociated complex 90 and the solvent 72) accommodated in the reaction chamber 10. Alternatively, the invention is not limited thereto, and the dissociation light DL may be radiated to a part of the content while the content is agitated. Incidentally, since such dissociation is irreversible, the dissociated complex 90 does not bind again to the first carrier 73 in the reaction chamber 10. Subsequently, the first carrier 73 after dissociation is removed from the reaction chamber 10. A method of removing the first carrier 73 after dissociation may be any method, however, for example, in a state in which the first carrier 73 after dissociation is magnetically collected by a magnet (not illustrated) installed in a part of the reaction chamber 10 (as an example, a side surface portion of the reaction chamber 10, etc.), the first carrier 73 may be removed by transferring the dissociated complex 90 and the solvent 72 to another reaction chamber 10.

### (Measuring method - pass-through step)

Next, the pass-through step is described. The pass-through step is performed in a procedure below to pass the dissociated complex 90 formed in the dissociating step through the pore 41 of the substrate 40. In more detail, first, predetermined amount of liquid comprising the dissociated complex 90 and the solvent 72 is removed from the reaction chamber 10 by the liquid feeding pipe, and the dissociated complex 90 and solvent 72 in the liquid are injected into the accommodating portion 30 of the detection mechanism (specifically, into the upper accommodating portion 32 illustrated in Fig. 8(a)). Subsequently, as illustrated in Fig. 8(b), a current C is caused to flow for a predetermined time through the accommodating portion 30 via the first electrode portion and the second electrode portion by a measurement unit of the control device to move the injected dissociated complex 90 downward (that is, electrophoresis is used), thereby passing the dissociated complex 90 through the pore 41. It is preferable that the predetermined time is set to a time longer than or equal to a generally assumed time required for the predetermined number of dissociated complexes 90 to pass through the pore 41. Alternatively, it is possible to adopt a scheme in which a time is set in advance (for example, 10 minutes) regardless of the number of dissociated complexes 90 passing through, and the dissociated complexes 90 passing through the pore 41 within the set time are analyzed.

### (Measuring method - measuring step)

Next, the measuring step is described. The measuring step is performed in a procedure below to measure a size of the dissociated complex 90. In more detail, first, by the measurement unit of the control device, detection of the current C flowing through the accommodating portion 30 by the detector is started at a timing at which the current C starts to flow in the pass-through step, and detection of the current C is continued until the predetermined time during which the current C flows elapses (incidentally, this operation is repeatedly performed until the pass-through step is finished). Subsequently, the measurement unit of the control device determines the size of each dissociated complex 90 passing through the pore 41 in the pass-through step (that is, measures the size of the dissociated complex 90) based on the detected change in the current C with reference to relationship data which is stored in a storage unit in advance and indicates a relationship between the size of the dissociated complex 90 and the magnitude of the current C (for example, data indicating a proportional relationship between the size of the dissociated complex 90 and the magnitude of the current C, etc.).

### (Measuring method - determining step)

Next, the determining step is described. The determining step is performed in a procedure below to determine the number of measurement targets 71 contained in the sample 70. In more detail, first, a determination unit of the control device determines the number of measurement targets 71 contained in the sample 70 based on the size of the dissociated complex 90 measured in the measuring step. A method of determining the number of measurement targets 71 contained in the sample 70 may be any method, however, in the second embodiment, a dissociated complex 90 (specifically, a first dissociated complex 91) whose size measured in the measuring step is greater than or equal to a threshold value (for example, 40 nm or more, etc.) stored in the storage unit in advance can be determined from among dissociated complexes 90 passing through the pore 41 in the pass-through step, and a integrated value of the number of determined first dissociated complexes 91 can be determined as the number of measurement targets 71 contained in the sample 70. A reason therefor is that since two types of dissociated complexes 90 (that is, the first dissociated complex 91 and the second dissociated complex 92 illustrated in Fig. 7(b)) are formed in the dissociating step, the first dissociated complex 91 can be determined from the first dissociated complex 91 and the second dissociated complex 92 using one threshold value even when the complex table 55a according to the first embodiment is not used. Subsequently, the controller of the control device causes the output unit to output the determined number of measurement targets 71 contained in the sample 70.

According to the measuring method described above, when compared to a conventional technology, the number of dissociated complexes 90 is measured based on the size of the dissociated complex 90 rather than measuring the number of fluorescent substances. Thus, for example, even when a plurality of first dissociated complexes 91 or a plurality of second dissociated complexes 92 is passed through the pore 41, it is possible to accurately measure only the number of first dissociated complexes 91 (that is, measurement targets 71 bound to the second carrier 76 by a specific reaction). Therefore, it is possible to improve accuracy of measurement. In addition, since the size of the dissociated complex 90 is less likely to vary than the size of the complex 78, it is easier to determine the number of measurement targets 71 in the determining step, so that it is possible to further improve accuracy of measurement.

According to the second embodiment, the complex 78 includes the measurement target 71, the first carrier 73, and the second carrier 76, and the dissociating step of forming the dissociated complex 90 by dissociating the first carrier 73 from the complex 78 is included before the pass-through step. Further, the dissociated complex 90 formed in the dissociating step is passed through the pore 41 in the pass-through step, the size of the dissociated complex 90 is measured in the measuring step based on the change in electrical characteristics occurring when the dissociated complex 90 is passed through the pore 41 in the pass-through step, and the number of measurement targets 71 is determined in the determining step based on the size of the dissociated complex 90 measured in the measuring step. Therefore, when compared to a conventional technology, the number of dissociated complexes 90 is measured based on the size of the dissociated complex 90 rather than measuring the number of fluorescent substances. Thus, for example, even when a plurality of dissociated complexes 90 (that is, the measurement target 71 bound to the second carrier 76 by the specific reaction) and a plurality of carriers nonspecifically bound only to the probe molecules is passed through the pore 41, it is possible to accurately measure the number of dissociated complexes 91. Therefore, it is possible to improve accuracy of measurement.

### [III] Modifications to embodiments

Even though the embodiments of the invention have been described above, specific configurations and means of the invention can be arbitrarily modified and improved within the scope of the technical idea of each invention described in the claims. Hereinafter, such modifications are described.

### (With regard to problems to be solved and effects of the invention)

First, problems to be solved and effects of the invention are not limited to the above-described content, and a problem not described above may be solved and an effect not described above may be obtained by the invention. In addition, a part of a described problem may be solved, and a part of a described effect may be obtained. For example, even when speed and simplicity of measurement work of the measuring method according to the invention are the same as those of a conventional method, in the case of having the same speed and simplicity of measurement work as those of the conventional method by a different method from the conventional method, the problem of the invention is solved.

### (With regard to dispersion and integration)

In addition, each of the above-described electrical components is functionally conceptual and is not necessarily physically configured as illustrated in the figure. In more detail, a specific mode of dispersion and integration of each unit is not limited to that illustrated in the figure, and all or a part thereof can be configured by being functionally or physically dispersed or integrated in any units according to various loads, usage situations, etc. For example, the control device may be dispersedly configured in a plurality of devices configured to be able to communicate with each other, the controller may be provided in a part of the plurality of devices, and the storage unit may be provided in another part of the plurality of devices.

### (With regard to shape, numerical value, structure, and time series)

With regard to components illustrated in the embodiments or the figures, a shape, a numerical value, or an interrelation of structures or time series of a plurality of components can be arbitrarily modified and improved within a range of a technical idea of the invention.

### (With regard to second carrier)

In the first and second embodiments, it is described that the second carrier 76 is formed to be smaller than the first carrier 73. However, the invention is not limited thereto. For example, the second carrier 76 may have a size greater than or equal to that of the first carrier 73.

### (With regard to passing-through portion)

In the first embodiment, it is described that the passing-through portion includes the first electrode portion and the second electrode portion. However, the invention is not limited thereto. For example, a pressurizing portion may be provided instead of or in addition to the first electrode portion and the second electrode portion, and the complex 78 and the solvent 72 accommodated in the upper accommodating portion 32 of the accommodating portion 30 may be pressurized downward using the pressurizing portion in the pass-through step. Alternatively, instead thereof or in addition thereto, salt concentration of the solvent may be made different between the accommodating portion 30 and the upper accommodating portion 32, or a concentration and separation step using capillary electrophoresis may be added (incidentally, the same is applied to the second embodiment). In particular, when a combination of the above means is used, it is possible to effectively pass the complex 78 through the pore 41.

### (With regard to measuring method)

In the first and second embodiments, it is described that the first reaction step and the second reaction step are performed. However, the invention is not limited thereto. For example, when the complex 78 formed in advance is used, the first reaction step and the second reaction step may be omitted.

### (With regard to measuring step)

In the first embodiment, the size of each complex 78 passing through the pore 41 in the pass-through step is determined based on the change in the current C (time history change in the current C) detected by the detector with reference to the relationship data indicating the relationship between the size of the complex 78 and the magnitude of the current C. However, the invention is not limited thereto. For example, even for the same complex 78, the size of the complex 78 is different depending on the scheme in which the complex 78 passes through the pore 41 (for example, a passing direction of the complex 78 is a vertical direction or a non-vertical direction, a plurality of complexes 78 passes continuously or intermittently, etc.). Therefore, for example, the size of the complex 78 measured in the measuring step may be corrected based on the change in the current C (time history change in the current C) detected by the detector and pattern data obtained by machine learning, etc., of the change in the current C for each scheme in which the complex 78 passes through the pore 41. As an example, in pattern data of the change in the current C, when the change in the current C corresponding to a predetermined complex 78 passing through the pore 41 in the pass-through step matches a pattern in which the passing direction of the complex 78 is the vertical direction, the size of the complex 78 is not corrected. However, when the change in the current C matches a pattern in which the passing direction of the complex 78 is the non-vertical direction, the size of the complex 78 is corrected.

### (With regard to dissociating step)

In the second embodiment, the dissociation light DL is radiated to the complex 78 formed in the second reaction step from the dissociation portion 80 to cleave the photo-cleavable linker, thereby dissociating the first carrier 73 from the complex 78. However, the invention is not limited thereto. Instead of the photo-cleavable linker, it is possible to select various linkers cut by a drug, an enzyme, a protein denaturing agent (such as urea), heat, etc. Further, it is possible to appropriately select dissociating means associated with a used linker. For example, in place of the dissociation portion 80 (or in combination with the dissociation portion 80), a drug capable of cleaving a linker (for example, a high concentration inorganic salt, a reducing agent dithiothreitol, 2-mercaptoethanol, etc.) may be added to the complex 78 and the solvent 72, or an enzyme (for example, a proteolytic enzyme, a sugar chain cleaving enzyme, a fatty acid decomposing enzyme, a nuclease, etc.) or urea capable of cleaving a linker may be added to the complex 78 and the solvent 72. As a linker cleavable by an enzyme, for example, a linker described in Japanese Patent No. 3287249, etc. is known. Alternatively, instead of radiating the dissociation light DL from the dissociation portion 80, it is possible to radiate heat set to a temperature at which the first carrier 73 is dissociated from the complex 78.

### [Examples]

### Example 1: Measurement of methylated DNA by pore sensing (first embodiment)

### (1) Preparation of streptavidin-bound magnetic particles (first carrier)

100 µL of 50 mg/mL ethyl(dimethylaminopropyl) carbodiimide and 100 µL of 50 mg/mL N-hydroxysuccinimide were added to 6 mg of magnetic particles (nanomag-D, COOH, 130 nm, product number: 09-02-132 manufactured by micromod Partikeltechnologie GmbH) suspended in 200 µL of 50 mM MES (pH = 5.5), and inverted and mixed at room temperature for 30 minutes. After performing washing twice with 200 µL of 50 mM MES (pH = 5.5), 120 µL of 1.0 mg/mL streptavidin (manufactured by Wako Pure Chemical Industries, Ltd., product number: 141-12851) dissolved in 50 mM MES (pH = 5.5), and inverted and mixed at room temperature for 30 minutes, thereby streptavidin bound on the magnetic particles. Streptavidin-bound magnetic particles were obtained by performing washing twice with 200 µL of 50 mM MES (pH = 5.5) and three times with 400 µL of TBS containing 2% BSA.

### (2) Preparation of anti-methylcytosine antibody-bound gold nanoparticles (second carrier)

For the anti-methylcytosine antibody 1G3 obtained by FUJIREBIO, INC., antibody-bound gold nanoparticles were prepared by a method described in a package insert using NHS-Activated Gold Nanoparticle Conjugation Kit (product number: CGN5K-40-1) manufactured by Cytodiagnostics Inc.

### (3) Measurement of methylated DNA (measurement target)

As a measurement target, methylated DNA which has a sequence of 5'-GCC XGC AXG TCC TXG XGG-3' (X is 5-methyl cytosine and the 5' end is biotin labeled) and is artificially synthesized by Hokkaido System Science Co., Ltd. was used. First, streptavidin-bound magnetic particles (6.25 µg) and a nucleic acid to be measured (500 fmol) were suspended in 75 µL of PBS and then allowed to react at 37°C for 30 minutes to form a complex of the streptavidin-bound magnetic particles and the nucleic acid to be measured. After performing washing with 200 µL of Lumipulse washing solution (manufactured by FUJIREBIO INC.), 100 µL of 1.2 × 10¹¹ NPS/mL anti-methylcytosine antibody-bound gold nanoparticle solution was added and allowed to react at 37°C for 2.5 hours. After performing washing with 200 µL of Lumipulse washing solution (self-manufactured), suspension in 250 µL of PBS was performed, and a particle size distribution was measured using a nanoparticle multi-analyzer (qNano manufactured by Izon Science Ltd.) (first specimen).

In addition, a solution not containing the nucleic acid to be measured was used, and a sample in which no complex was formed was similarly measured (second specimen).

### (4) Result

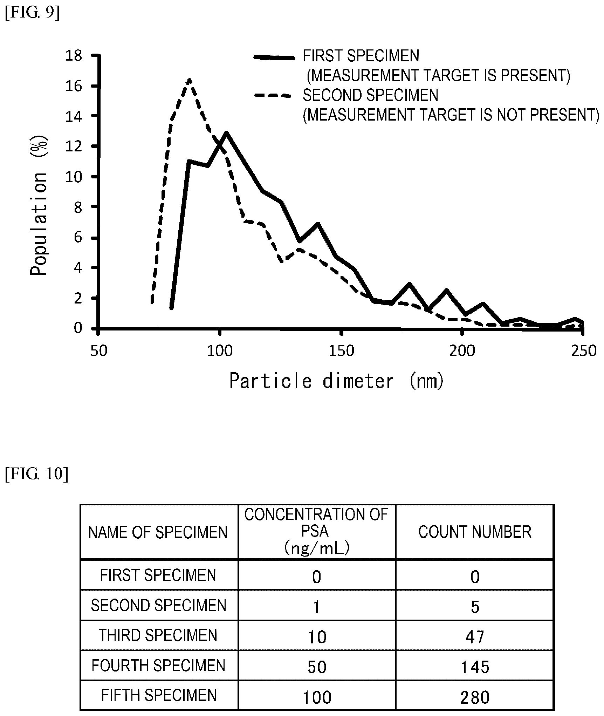

Fig. 9 shows a graph showing an experimental result for each specimen size related to the first specimen and an experimental result for each specimen size related to the second specimen, where a horizontal axis represents a measured value (indicated as "particle diameter (nm)" in Fig. 9) of a size of a specimen, and a vertical axis represents a value obtained by dividing a sum of the number of specimens for each specimen size by the number (total number) of specimens contained in the sample 70 (indicated as "population (%)" in Fig. 9). As shown in Fig. 9, from these experimental results, it was confirmed that the divided value of the first specimen for each specimen size is different from the divided value of the second specimen. In addition, it was also confirmed that the divided value of the second specimen shows a tendency to become higher than the divided value of the first specimen as the size of the specimen becomes larger. As described above, it was found from the experimental results shown in Fig. 9 that the number of magnetic particles and the number of complexes can be distinguished and measured, and effectiveness of the measuring method according to the first embodiment could be confirmed.

### Example 2: Measurement by pore sensing of PSA (second embodiment)

### (1) Preparation of photo-cleavable linker-bound anti-PSA antibody

NHS-PC-Biotin (manufactured by AmberGen, Inc., product number PCB-N-005) was bound to an anti-PSA antibody (No. 62, self-manufactured) using a method described in a package insert to obtain a photo-cleavable linker-bound anti-PSA antibody.

### (2) Preparation of anti-PSA antibody-bound magnetic particles (photo-cleavable linker) (first carriers)

Streptavidin-bound magnetic particles were prepared in the same manner as in Example 1 (1). 2.7 µg of a photo-cleavable linker-bound anti-PSA antibody was added to 1 mg of the streptavidin-bound magnetic particles and inverted and mixed at 37°C for 30 minutes, to bind the photo-cleavable linker-bound anti-PSA antibody on the magnetic particles. The anti-PSA antibody-bound magnetic particles (photo-cleavable linkers) were obtained by performing washing three times with 10 mM Tris buffer solution.

### (3) Preparation of anti-PSA antibody-bound fluorescent nanoparticles (second carriers)

2.2 µL of 10.0 mg/mL 4- (4,6 - dimethoxy -1,3,5- triazin -2- yl) -4-methylmorpholinium chloride was added to 2.5 mg of fluorescent nanoparticles (Fluospheres, COOH, 40 nm, product number F8789, manufactured by Life Technologies Corporation) and inverted and mixed at room temperature for 180 minutes. 40 µL of 3.0 mg/mL anti-PSA antibody (No. 31, self-manufactured) dissolved to 100 mM sodium bicarbonate aqueous solution was added, and inverted and mixed at room temperature for 60 minutes to bind the anti-PSA antibody to the fluorescent nanoparticles. After addition of 100 µL of 190 mM glycine aqueous solution, supernatant was removed by centrifugation to obtain anti-PSA antibody-bound fluorescent nanoparticles.

### (4) Measurement of PSA (measurement target)

Lumipulse PSA standard solution (manufactured by FUJIREBIO INC.) was diluted with PBS as needed, and PSA solutions of 0, 1, 10, 50 and 100 ng/mL (first, second, third, fourth and fifth test specimens) were prepared. 20 µL of each specimen and 250 µL of a solution containing 0015% anti-PSA antibody-bound magnetic particles (photo-cleavable linkers) were allowed to react at 37°C for 10 minutes, and PSA antigen was captured on the anti-PSA antibody-bound magnetic particles (photo-cleavable linkers). After washing non-bound substances with 750 µL of Lumipulse washing solution (manufactured by FUJIREBIO INC.), a solution containing 250 µL of 32 µg/mL anti-PSA antibody-bound fluorescent nanoparticles was added and allowed to react at 37°C for 10 minutes. After performing washing with 500 µL of Lumipulse washing solution, suspension in 100 µL of PBS was performed, and 365 nm of UV light (C11924-111 and L11922-401 manufactured by Hamamatsu Photonics K.K.) was irradiated for one minute (dissociating step). The supernatant from which the magnetic particles were removed was measured using the nanoparticle multi-analyzer (qNano manufactured by Izon Science Ltd.), and a particle count number for 10 minutes was measured.

### (5) Effect

Fig. 10 is a table showing experimental results for a first specimen to a fifth specimen. From the experimental results in Fig. 10, it was confirmed that a significant difference had occurred between the number (count number) of first specimens (without PSA (measurement target)) and the count numbers of the second specimen to the fifth specimen (with PSA). In addition, it was confirmed that the count number of PSA tends to increase as the concentration of PSA increases. As described above, it is found from the experimental results shown in Fig. 10 that the count number of the measurement target varies depending on the concentration of the measurement target, and effectiveness of the measuring method according to the second embodiment could be confirmed.

### Reference Signs List

- 1: Measuring system
- 10: Reaction chamber
- 20: Detection mechanism
- 30: Accommodating portion
- 31: Lower accommodating portion
- 32: Upper accommodating portion
- 40: Substrate
- 41: Pore
- 50: Control device
- 51: Operation unit
- 52: Output unit
- 53: Power supply unit
- 54: Controller
- 54a: Measurement unit
- 54b: Determination unit
- 55: Storage unit
- 55a: Complex table
- 70: Sample
- 71: Measurement target
- 72: Solvent
- 73: First carrier
- 74: First carrier-side antibody
- 75: Second carrier-side antibody
- 76: Second carrier
- 78: Complex
- 80: Dissociation portion
- 90: Dissociated complex
- 91: First dissociated complex
- 92: Second dissociated complex
- C: Current
- DL: Dissociation light

## Claims

1. A measuring method of measuring a measurement target contained in a sample, the method comprising:
a pass-through step: passing a complex formed by allowing the measurement target to react with a predetermined carrier through a pore provided in a substrate;
a measuring step: measuring a size of the complex based on change in electrical characteristics occurring when the complex is passed through the pore in the pass-through step; and
a determining step: determining the number of measurement targets based on the size of the complex measured in the measuring step.

2. The measuring method according to claim 1,
wherein the complex comprises the measurement target, a first carrier capable of binding to the measurement target, and a second carrier capable of binding to the measurement target, and
in the determining step, the number of measurement targets for each complex passing through the pore in the pass-through step is determined based on the size of the complex measured in the measuring step, and the integrated value of each determined number is determined as the total number of measurement targets.

3. The measuring method according to claim 1,
wherein the complex comprises the measurement target, a first carrier capable of bind to the measurement target, and a second carrier capable of bind to the measurement target,
the measuring method further comprises a dissociating step of forming a dissociated complex by dissociating the first carrier from the complex before the pass-through step,
the dissociated complex formed in the dissociating step is passed through the pore in the pass-through step,
a size of each dissociated complex is measured in the measuring step based on change in electrical characteristics occurring when the dissociated complex is passed through the pore in the pass-through step, and
the number of measurement targets is determined in the determining step based on the size of each dissociated complex measured in the measuring step.

4. A measuring system for measuring a measurement target contained in a sample, the measuring system comprising:
a substrate in which a pore is formed;
pass-through means for passing through to the pore a complex formed by allowing the measurement target to react with a predetermined carrier;
measuring means for measuring a size of the complex based on change in electrical characteristics occurring when the complex is passed through the pore by the pass-through means; and
determining means for determining the number of measurement targets based on the size of each complex measured by the measuring means.

5. The measuring system according to claim 4,
wherein the complex comprises the measurement target, a first carrier capable of binding to the measurement target, and a second carrier capable of binding to the measurement target, and
the measuring system further comprises complex information storage means for storing complex information configured by mutually associating an information indicating the size of the complex and number-of-measurement targets information indicating the number of measurement targets with each other, and
the determining means determines the number of measurement targets of each complex passed through the pore by the pass-through means based on the complex information stored in the complex information storage means and the size of the complex measured by the measuring means, and determines an integrated number of each determined number as the number of measurement targets.

6. The measuring system according to claim 4,
wherein the complex comprises the measurement target, a first carrier capable of binding to the measurement target, and a second carrier capable of binding to the measurement target,
the measuring system further comprises dissociating means for forming a dissociated complex by dissociating the first carrier from the complex,
the pass-through means passes the dissociated complex formed by the dissociating means through the pore,
the measuring means measures a size of the dissociated complex based on change in electrical characteristics occurring when the dissociated complex is passed through the pore by the pass-through means, and
the determining means determines the number of measurement targets based on the size of the dissociated complex measured by the measuring means.
